# FASCICULE DE BREVET EUROPEEN

(11) **EP 1 920 809 B1**
(45) Date de publication et mention de la délivrance du brevet: **24.12.2014**
(21) Numéro de dépôt: 07291266.0
(22) Date de dépôt: 17.10.2007
(51) Int. Cl.: B01D 1/28, B01D 3/00, B01D 53/26, C07C 29/76

(54) **Nouveau procédé de déshydratation d'alcool et installation pour sa mise en oeuvre**
Neuartiges Verfahren zur Dehydratisierung von Alkohol und Anlage für dessen Umsetzung
New method for dehydrating alcohol and installation for implementing same

(30) Priorité: 17.10.2006 FR 0609090
(43) Date de publication de la demande: 14.05.2008
(73) Titulaire: MAGUIN S.A., F-02800 Charmes (FR)
(72) Inventeur: Doreau, Geneviève, 94320 Thiais (FR); Touron, Jerôme, 75013 Paris (FR)
(74) Mandataire: Hirsch & Associés

(56) Documents cités:
- EP-A1- 0 306 358
- WO-A-2004/088230
- FR-A1- 2 719 039
- FR-A1- 2 855 170
- US-A- 4 405 409
- US-A- 4 407 662

## Description

### DOMAINE TECHNIQUE

La présente invention a pour objet un nouveau procédé de déshydratation d'alcool par adsorption sur tamis moléculaire, qui offre une économie d'énergie par rapport aux procédés et installations existants. L'invention a aussi pour objet la mise en oeuvre du nouveau procédé selon l'invention.

### ARRIERE-PLAN TECHNIQUE

On connaît de façon générale des procédés de déshydratation d'alcool. Parmi ces procédés de déshydratation d'alcool, certains utilisent la distillation azéotropique, avec du cyclohexane comme tiers corps. Cependant, cette méthode est coûteuse en énergie et utilise du cyclohexane, composé toxique.

Un procédé de déshydratation par pervaporation au travers de membranes a également été proposé. Cependant, comme les membranes utilisées sont fragiles, il se pose des problèmes d'échelle avec ce type de procédé. Par ailleurs, le contrôle des conditions opératoires est difficile.

Il a aussi été proposé un procédé de déshydratation d'éthanol sur tamis moléculaires qui permet, par rapport au procédé de distillation azéotropique, d'économiser de l'énergie et d'éviter l'emploi de cyclohexane. Dans un procédé de ce type, il est nécessaire de régénérer le tamis moléculaire sur lequel est adsorbée l'eau. Cette régénération fait appel principalement à deux techniques, dénommées TSA et PSA, respectivement Temperature Swing Adsorption et Pressure Swing Adsorption.

Le procédé TSA fait appel au fait que les quantités de gaz adsorbés diminuent lorsque la température augmente, pour une pression donnée. Pour effectuer la régénération thermique d'un tamis moléculaire, on envoie un courant de fluide gazeux chaud, air ou gaz inerte, dans le ballon contenant le tamis. Ceci a pour effet de refouler le produit adsorbé vers l'entrée de la colonne.

Le procédé PSA fait appel au fait que la désorption peut être obtenue en appliquant une forte diminution de pression. Ce second procédé est principalement utilisé dans le domaine de la purification des gaz industriels.

Le procédé TSA n'est pas facilement transposable à la déshydratation des alcools, car il nécessite l'emploi de gaz, tels que l'azote, l'argon, le dioxyde de carbone et autres, qui sont ensuite rejetés dans l'atmosphère. Ceci implique donc la présence de cuves de stockage, qui sont d'un coût élevé et présente des problèmes inhérents aux cuves de stockage, notamment de sécurité.

Une amélioration à ces procédés a été proposée dans le brevet US 4 407 662. Celui-ci décrit un procédé de déshydratation d'un mélange eau/éthanol par adsorption/régénération sur tamis moléculaires, comprenant les étapes de (i) chauffage du mélange eau/éthanol jusqu'à la phase vapeur, à une température comprise entre 90 et 120°C et à une pression comprise entre 2 et 10 psig ; (ii) passage du mélange eau/éthanol en phase vapeur sur un tamis moléculaire pour provoquer l'adsorption de l'eau sur ledit tamis moléculaire ; (iii) passage d'une partie de l'éthanol déshydraté, à une pression inférieure à la pression atmosphérique, sur un tamis moléculaire chargé en eau pour désorber l'eau adsorbée ; et (iv) inversion des étapes (ii) et (iii) au travers des deux tamis moléculaires.

Cependant, ce procédé ne donne pas entière satisfaction, et ce pour plusieurs raisons. Tout d'abord, l'étape de désorption du tamis moléculaire chargé en eau utilise environ 20% de l'éthanol déshydraté, ce qui représente une part significative, et de plus conduit finalement à un mélange eau/éthanol qu'il faut à nouveau purifier. Par ailleurs, il est difficile de contrôler cette étape de désorption, alors que des caractéristiques identiques pour les tamis doivent être obtenues avant chaque début du cycle d'adsorption. Afin de contrôler la température régnant dans le tamis pendant la phase de régénération, et aussi pour éviter une éventuelle condensation de l'eau pendant la phase d'adsorption, il a été proposé de munir le ballon contenant le tamis d'une double paroi afin de contrôler plus efficacement la température régnant dans le ballon. Cependant, cette solution n'est pas satisfaisante, notamment en raison des surcoûts engendrés.

Le document FR 2 719 039 propose encore une amélioration par rapport au procédé ci-dessus, consistant en l'adjonction, avant l'étape (iii) de régénération, d'une étape supplémentaire de surchauffage de la partie de l'éthanol déshydraté devant passer sur le tamis moléculaire pour désorber l'eau adsorbée. Cette amélioration permet un contrôle accru de la désorption et une réduction de la quantité d'éthanol déshydraté nécessaire pour la phase de désorption (d'environ 20 à environ 15 %).

Par ailleurs, le document CA 2 503 067 décrit un système de production d'alcool déshydraté à partir de végétaux, dans lequel un couplage énergétique est prévu entre l'unité de déshydratation d'alcool proprement dite et d'autres unités du système.

Néanmoins, le niveau de consommation d'énergie de tous les procédés de l'art antérieur décrits ci-dessus reste problématique, en particulier si l'on considère que l'alcool déshydraté est principalement destiné à la production d'alcool carburant, qui est en concurrence avec les procédés de raffinage du pétrole. Il est donc souhaitable de parvenir à optimiser davantage la consommation en énergie primaire des procédés industriels de production d'alcool déshydraté et en particulier de parvenir à optimiser la consommation énergétique spécifiquement au niveau de l'unité de déshydratation d'alcool.

### RESUME DE L'INVENTION

L'invention a donc pour objet un procédé de déshydratation d'un mélange eau / alcool comprenant les étapes suivantes :
(i) vaporisation et surchauffe du mélange eau / alcool, à une température suffisante pour maintenir ledit mélange à l'état vapeur au cours de l'étape (ii) ;
(ii) adsorption par passage du mélange eau / alcool à l'état vapeur obtenu à l'étape (i) sur un tamis moléculaire pour provoquer l'adsorption de l'eau sur ledit tamis moléculaire, permettant d'obtenir de la vapeur d'alcool déshydraté ;
(iii) condensation de la vapeur d'alcool déshydraté obtenue à l'étape (ii), permettant de récupérer de l'énergie,
   dans lequel la vaporisation et / ou la surchauffe du mélange eau / alcool de l'étape (i) sont effectuées au moins partiellement grâce à l'énergie récupérée à l'étape (iii) ; et dans lequel :
   - la condensation de l'étape (iii) est précédée d'une compression mécanique de la vapeur d'alcool déshydraté ; ou
   - la condensation de l'étape (iii) est couplée à la vaporisation d'un fluide caloporteur et le fluide caloporteur vaporisé subit une compression.

Selon un mode de réalisation, l'alcool est l'éthanol.

Selon un mode de réalisation, le mélange eau / alcool contient au moins 80 % en volume d'alcool, de préférence au moins 90 % en volume d'alcool, de préférence au moins 92 % en volume d'alcool.

Selon un mode de réalisation, le procédé selon l'invention comprend, concurremment et / ou alternativement aux étapes (i) à (iii), l'étape supplémentaire suivante :
(iv) régénération par passage d'une partie de l'alcool déshydraté, à une pression inférieure à la pression atmosphérique, sur un tamis moléculaire saturé en eau pour désorber l'eau adsorbée,
ladite étape produisant un effluent.

Selon un mode de réalisation, le procédé selon l'invention comprend à l'étape (iv) un surchauffage de la partie de l'alcool déshydraté devant passer sur le tamis moléculaire saturé en eau pour désorber l'eau adsorbée.

Selon un mode de réalisation, l'effluent obtenu à l'étape (iv) est combiné avec le mélange eau / alcool à traiter.

Selon un mode de réalisation, deux, trois tamis moléculaires ou plus opèrent selon un mode en alternance.

Selon un mode de réalisation, le procédé selon l'invention nécessite une consommation de vapeur d'eau inférieure à environ 50 kg pour 100 litres d'alcool déshydraté produit.

L'invention a également pour objet une installation pour la déshydratation d'un mélange eau / alcool, comprenant :
- une conduite d'amenée du mélange eau / alcool à traiter ;
- une unité de vaporisation pour assurer la vaporisation du mélange circulant dans la conduite précitée ;
- un surchauffeur pour surchauffer la vapeur issue de l'unité de vaporisation ;
- un ou plusieurs ballons connectés au surchauffeur et contenant un tamis moléculaire ;
- une unité de condensation de l'alcool déshydraté, en aval des ballons et assurant une partie de l'alimentation en énergie de l'unité de vaporisation ;
- une ou des conduites de collecte de l'alcool déshydraté connectées en sortie de l'unité de condensation ;
   et comprenant en outre :
   - une unité de compression mécanique entre les ballons et l'unité de condensation ; ou
   - un circuit de fluide caloporteur, ledit circuit étant prévu pour permettre les échanges de chaleur entre le fluide caloporteur et, d'une part l'alcool déshydraté au niveau de l'unité de condensation, d'autre part l'eau ou le mélange eau / alcool au niveau de l'unité de vaporisation au moyen d'une injection directe ou au moyen d'un échangeur de chaleur, le circuit de fluide caloporteur comprend un système de thermo-éjection alimenté en vapeur à haute pression prévu pour recomprimer le fluide caloporteur à l'état vapeur.

Selon un mode de réalisation, l'unité de vaporisation comprend une colonne de distillation.

Selon un mode de réalisation, l'unité de condensation est prévue pour évaporer directement le mélange eau / alcool au niveau de l'unité de vaporisation.

Selon un mode de réalisation, l'installation selon l'invention comprend en outre un échangeur de chaleur assurant une partie de l'alimentation en énergie de l'unité de vaporisation.

Selon un mode de réalisation, l'installation selon l'invention comprend en outre :
- des conduites de désorption pour ramener une partie de l'alcool déshydraté vers les ballons ;
- des conduites de soutirage de l'effluent issu des ballons après régénération.

Selon un mode de réalisation, l'installation selon l'invention comprend en outre une conduite de recyclage destinée à recycler l'effluent vers l'unité de vaporisation.

Selon un mode de réalisation, l'installation selon l'invention comprend en outre une conduite de recyclage destinée à recycler l'effluent vers une unité de distillation alimentant en vapeur un échangeur de chaleur destiné à fournir de l'énergie à l'unité de vaporisation.

Selon un mode de réalisation, l'installation selon l'invention comprend en outre un moyen de chauffage placé au contact des conduites de désorption.

Par rapport à l'art antérieur discuté ci-dessus, le procédé selon l'invention permet une plus grande économie d'énergie, impliquant une économie de coûts et une limitation des nuisances environnementales. En effet, le procédé selon l'invention permet la récupération (le recyclage) de l'énergie contenue dans les vapeurs d'alcool déshydraté produites lors de l'étape d'adsorption afin de diminuer la consommation énergétique globale du procédé de déshydratation d'alcool. Certes, ce recyclage énergétique ne suffit pas à lui seul à vaporiser et surchauffer le mélange eau / alcool, et des moyens complémentaires de vaporisation et de surchauffage sont prévus dans l'invention. Mais il faut noter que, le coût de production de la vapeur d'eau étant plus élevé que le coût de l'électricité, il est particulièrement intéressant de proposer un procédé tel que celui de la présente invention, qui vise à réduire la consommation en vapeur d'eau. Le gain énergétique obtenu est de 30 % à 40 %.

Selon un mode de réalisation particulier, le procédé selon l'invention prévoit une recompression de vapeur (vapeur d'alcool déshydraté ou vapeur d'eau) particulièrement avantageuse en termes d'optimisation énergétique.

### BREVE DESCRIPTION DES FIGURES

La **figure 1****,** la **figure 1** **bis** et la **figure 1** **ter** représentent schématiquement trois types possibles d'installation selon l'invention, comprenant un système dit de thermo-compression.
La **figure 2** et la **figure 2** **bis** représentent schématiquement deux types possibles d'installation selon l'invention, comprenant un système dit de compression mécanique.

### DESCRIPTION DETAILLEE DE MODES DE REALISATION

La description qui suit concerne des modes de réalisations particuliers de l'invention et ne limite pas la portée générale de celle-ci.

### Installation à compression thermique

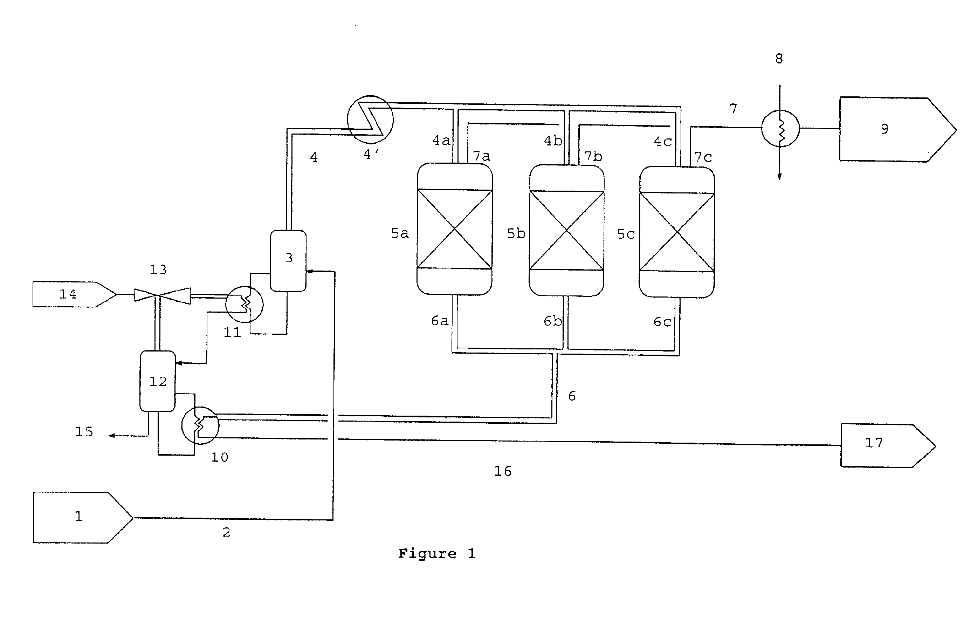

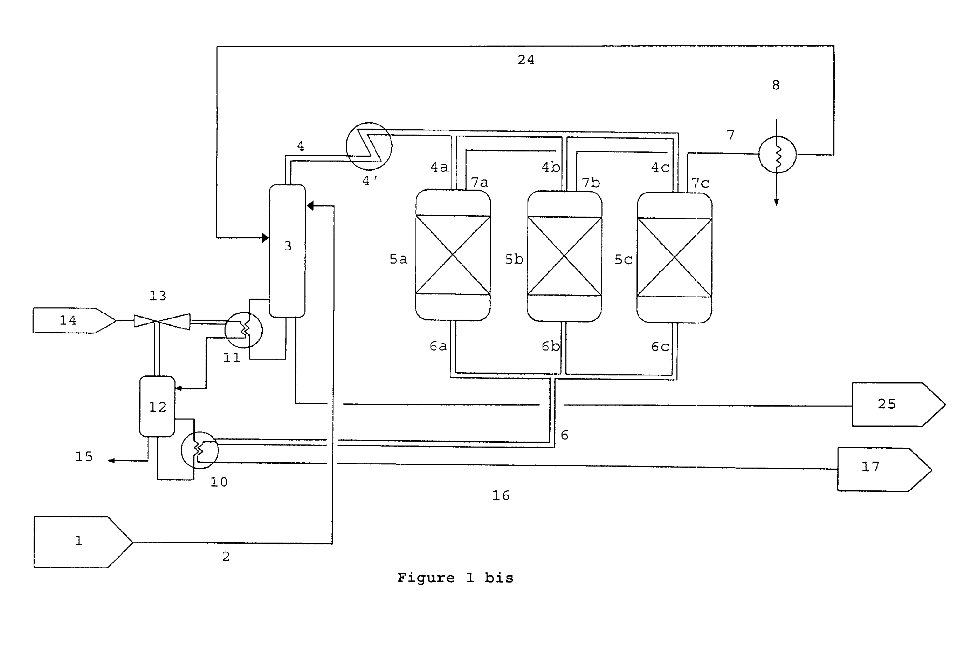

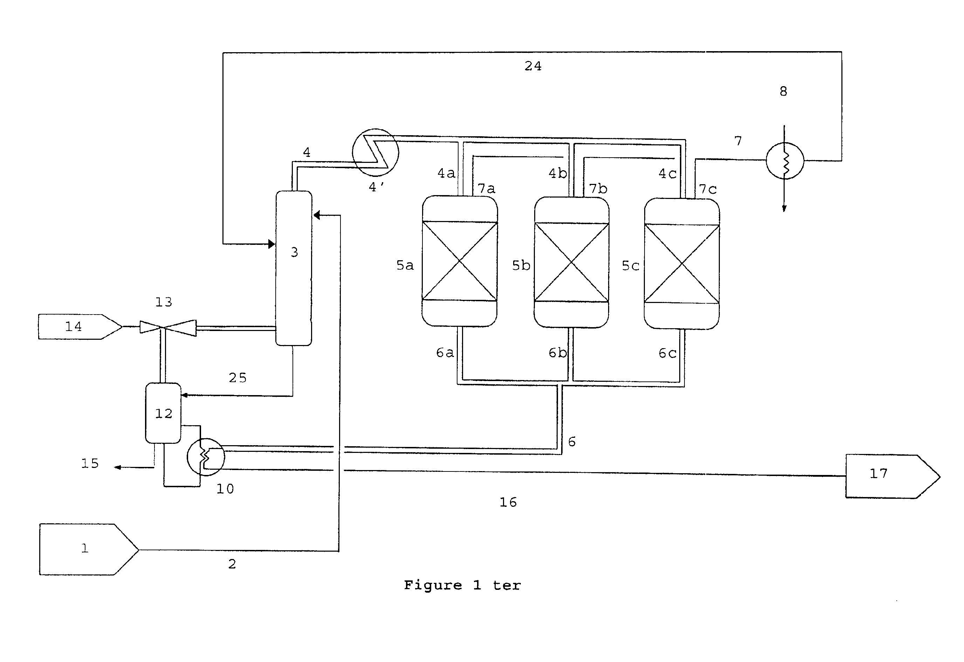

L'invention va à présent être décrite plus en détail en relation avec le mode de réalisation représenté à la **figure 1****.**

Un récipient de stockage 1 contient le mélange eau / alcool à purifier. Par récipient de stockage, on doit aussi comprendre en fait toute unité susceptible de fournir un mélange eau / alcool à traiter. En particulier, une unité installée dans une distillerie est considérée aux fins de l'invention comme un récipient de stockage. Une conduite d'amenée 2 relie le récipient de stockage 1 à une unité de vaporisation 3. Dans le cas de la **figure 1****,** cette unité de vaporisation 3 est un simple évaporateur. Ledit évaporateur est alimenté en chaleur par un échangeur de chaleur 11. En sortie de l'évaporateur est connectée une conduite 4, qui se subdivise en embranchements 4a, 4b, 4c. Un surchauffeur 4' est prévu au contact d'une partie de la conduite 4. Chaque embranchement 4a, 4b, 4c est connecté en entrée respectivement de ballons 5a, 5b, 5c comprenant chacun un tamis moléculaire.

Le tamis contenu dans les ballons est tout tamis approprié pour l'adsorption d'eau. A titre d'exemple, il peut présenter les caractéristiques suivantes : diamètre nominal des pores de 3 Å (0,3 nm). Un tamis approprié est notamment fourni par les zéolites synthétiques de structure cristalline de type A, par exemple celles disponibles chez CECA, La Défense, France, sous la référence Siliporite EPX 3B.

Un système de vannes (non représenté) assure le fonctionnement des ballons en alternance, de sorte que parmi les ballons 5a, 5b, et 5c, à un instant donné, deux fonctionnent par exemple en mode déshydratation et sont donc alimentés par le mélange de vapeur eau / alcool surchauffée, tandis que le troisième fonctionne en mode régénération.

Trois conduites de soutirage 6a, 6b et 6c convergeant vers une conduite principale de soutirage 6 sont prévues en sortie respectivement des ballons 5a, 5b, 5c. La conduite principale de soutirage 6 est connectée à une unité de condensation (ou échangeur de chaleur ou condenseur évaporateur) 10, en sortie duquel est branchée une conduite de collecte 16 qui est reliée à une zone de stockage et / ou de traitement 17 de l'alcool déshydraté.

L'installation comprend par ailleurs un circuit de fluide caloporteur. Par « fluide caloporteur », on entend un fluide tel que l'eau, éventuellement sous pression, ou des sels fondus, circulant dans un circuit distinct de celui du mélange eau / alcool et de l'alcool déshydraté, qui est susceptible de recevoir de l'énergie de la part de l'alcool déshydraté lors de la condensation de celui-ci (cet échange d'énergie impliquant généralement notamment la vaporisation du fluide caloporteur), et qui est susceptible de céder de l'énergie au mélange eau / alcool (cet échange d'énergie impliquant généralement notamment la condensation dudit fluide caloporteur).

En l'espèce, le circuit de fluide caloporteur comprend notamment un ballon d'évaporation 12 alimenté en chaleur par l'unité de condensation 10. La sortie vapeur du ballon d'évaporation 12 alimente un thermo-éjecteur 13, lequel est également alimenté par ailleurs par une source de vapeur à haute pression 14 (produite par une chaudière non représentée). La sortie du thermo-éjecteur 13 est connectée à l'échangeur de chaleur 11 qui chauffe l'unité de vaporisation 3. Le circuit du fluide caloporteur est complété par une conduite reliant en retour l'échangeur de chaleur 11 au ballon d'évaporation 12, ainsi que par une ligne 15 en sortie du ballon d'évaporation 12. La ligne 15 retourne vers la chaudière non représentée qui fournit la source de vapeur à haute pression 14.

Il faut par ailleurs noter que l'installation comprend des systèmes complémentaires de vannes, de pompes à vide et éventuellement de surchauffage pour faire fonctionner les ballons 5a, 5b et 5c en désorption. Des conduites de désorption (non représentées) peuvent être prévues pour ramener une partie de l'alcool déshydraté ver les ballons 5a, 5b, 5c, avec éventuellement un moyen de chauffage au contact de ces conduites de désorption. De plus, des conduites de soutirage de l'effluent 7a, 7b et 7c sont connectées respectivement à chaque ballon, et elles se rassemblent en une conduite de soutirage principale de l'effluent 7, qui après passage par une unité de refroidissement 8 (telle qu'une unité de refroidissement à eau), est reliée à une zone de traitement et / ou de stockage 9 de l'effluent ou éluat de désorption.

L'homme du métier saura aisément apporter un certain nombre d'adaptations à l'installation décrite ci-dessus. Par exemple, le nombre de ballons comprenant un tamis moléculaire peut varier et peut être égale à un, à deux ou à plus de trois.

Plus particulièrement, une variante possible d'installation à thermo-compression selon l'invention est donnée par le schéma **figure 1** **bis.** Selon ce schéma, l'unité de vaporisation 3 n'est pas un simple évaporateur (comme dans le cas de la **figure 1**) mais comprend au contraire une unité de distillation. La première particularité de cette variante est que l'unité de distillation comprend en bas de colonne de distillation une conduite de soutirage d'eau 25. Une autre particularité de cette variante est que la conduite de soutirage principale de l'effluent 7, après passage par l'unité de refroidissement 8, débouche sur une conduite de recyclage 24 qui alimente ladite unité de distillation en complément de l'alimentation via la conduite d'amenée 2.

Une autre variante encore est représentée à la **figure 1** **ter.** Selon cette variante, l'unité de vaporisation 3 comprend également une colonne de distillation, qui est alimentée en partie par la conduite de recyclage 24. Mais cette fois le circuit du fluide caloporteur est modifié et il se confond partiellement avec le circuit principal du mélange eau / alcool, puisque la colonne de distillation est intégrée au circuit du fluide caloporteur. En effet, le thermo-éjecteur 13 alimente directement le bas de la colonne de distillation, tandis que la conduite de soutirage d'eau 25 alimente en retour le ballon d'évaporation 12. L'échangeur de chaleur 11 est donc absent dans cette variante de l'installation.

### Installation à compression mécanique

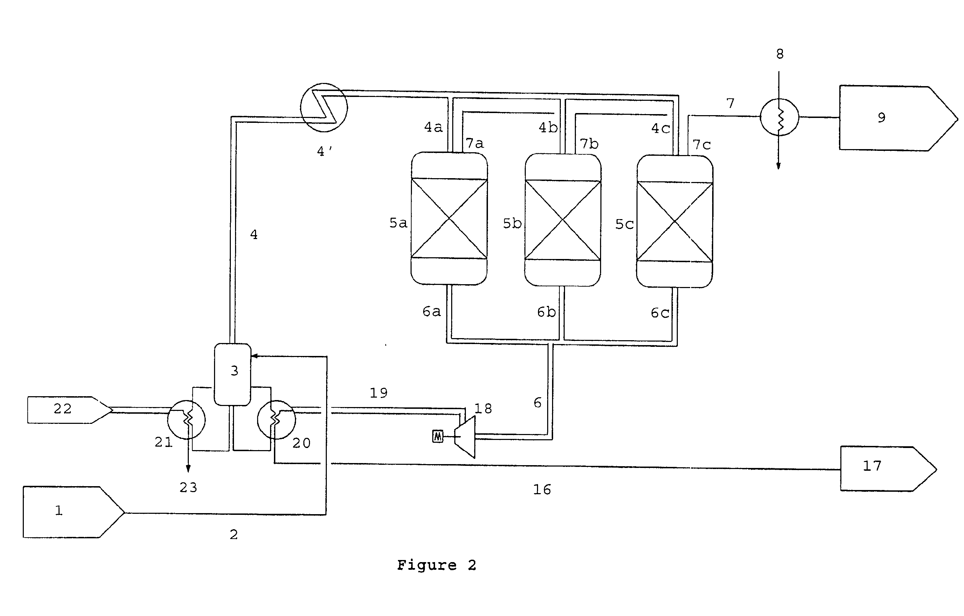

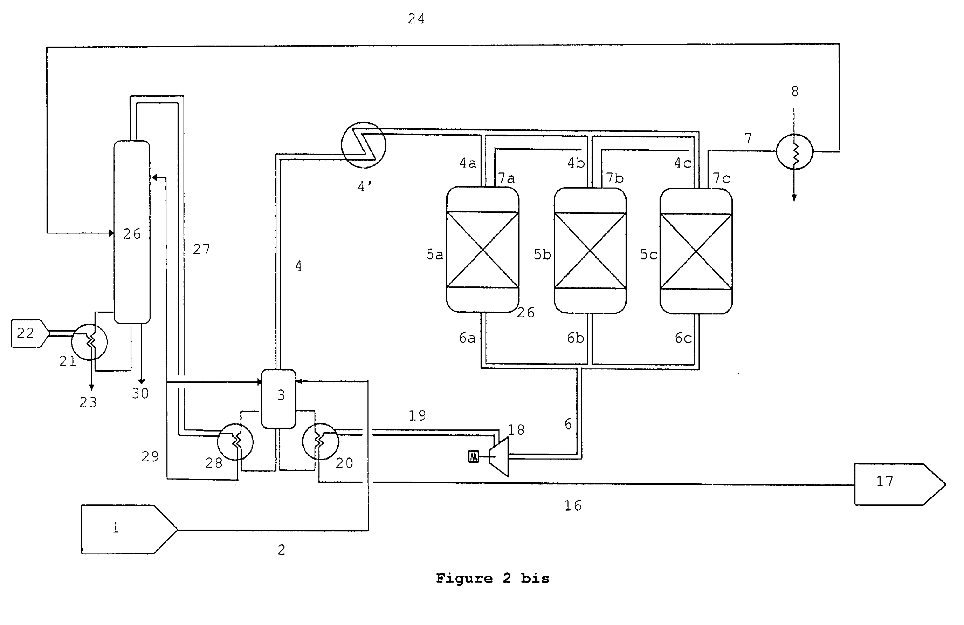

L'invention va à présent être décrite plus en détail en relation avec le mode de réalisation représenté à la **figure 2****.**

L'installation en question comprend un certain nombre d'éléments dont l'agencement est similaire à celui qui a été décrit en relation avec la **figure 1****,** à savoir : un récipient de stockage 1 ; une conduite d'amenée 2 ; une unité de vaporisation 3 (qui dans la **figure 2** est un simple évaporateur), en sortie duquel est branchée une conduite 4, se subdivisant en embranchements 4a, 4b, 4c ; un surchauffeur 4' au contact d'une partie de la conduite 4 ; des ballons 5a, 5b, 5c comprenant chacun un tamis moléculaire, alimentés respectivement par les embranchements 4a, 4b, 4c ; en sortie des ballons 5a, 5b, 5c, d'une part des conduites de soutirage 6a, 6b et 6c convergeant vers une conduite principale de soutirage 6 et d'autre part des conduites de soutirage de l'effluent 7a, 7b et 7c se rassemblant en une conduite de soutirage principale de l'effluent 7, reliée à une unité de refroidissement 8 puis à une zone de traitement et / ou de stockage 9 de l'éluat de désorption ou effluent.

Le système non représenté de fonctionnement en alternance des ballons et de basculement du mode déshydratation au mode désorption et réciproquement est similaire à celui de la **figure 1****.**

Toutefois, le système en question diffère de celui de la **figure 1** en ce que la conduite principale de soutirage 6 est branchée en entrée d'une unité de compression mécanique 18. En sortie de celle-ci, une conduite intermédiaire 19 alimente une unité de condensation 20 en sortie de laquelle est placée une conduite de collecte 16 reliée à une zone de stockage et / ou de traitement 17 de l'alcool déshydraté, tout comme dans le cas précédent. L'unité de condensation 20 fournit directement de la chaleur à l'unité de vaporisation 3. L'autre partie de l'alimentation en énergie de l'unité de vaporisation 3 provient d'un circuit auxiliaire comprenant un échangeur de chaleur 21, connecté en entrée à une source de vapeur à basse pression 22 et en sortie à une conduite de collecte de condensat 23.

Parmi les nombreuses variantes possibles de cette installation de compression mécanique, une alternative particulière est représentée à la **figure 2** **bis,** dans laquelle l'échangeur de chaleur 21 n'est pas directement connecté à l'unité de vaporisation 3.

En effet, cette solution alternative comprend un élément supplémentaire qui est une colonne de distillation 26. L'alimentation principale de cette colonne de distillation est assurée par la conduite de recyclage 24. Dans la partie inférieure de la colonne de distillation est branchée une conduite de soutirage d'eau 30, tandis qu'en tête de la colonne est branchée une conduite de soutirage de vapeur à basse pression 27, qui alimente un échangeur de chaleur 28. L'échangeur de chaleur assure le transfert de chaleur entre la conduite 27 et l'unité de vaporisation 3. Par ailleurs, une conduite de collecte de condensat 29, branchée en sortie de l'échangeur de chaleur 28 se divise en deux parties, dont l'une assure un retour vers la colonne de distillation 26, et dont l'autre alimente l'unité de vaporisation 3 (en complément de l'alimentation par la conduite d'amenée 2). Et c'est le chauffage de la colonne de distillation 26 (et non pas directement celui de l'unité de vaporisation 3) qui est assuré par le circuit auxiliaire composé de l'échangeur de chaleur 21, de la source de vapeur 22 et de la conduite de collecte de condensat 23.

### Procédé de déshydratation d'alcool

Le fonctionnement des installations décrites ci-dessus dans le cadre de la mise en oeuvre du procédé selon l'invention va à présent être détaillé.

### Etape (i)

Quel que soit le type d'installation retenue, le mélange eau / alcool à traiter est prélevé du récipient de stockage 1 via la conduite d'amenée 2 vers l'unité de vaporisation 3. Dans le cadre de la présente description, l'alcool est de l'éthanol. En effet, le procédé selon l'invention est en particulier destiné à être mis en oeuvre à partir de matières premières sur substrat sucré ou amylacé, dans le cadre de l'industrie chimique, et tout particulièrement dans le cadre de la production d'alcool carburant. Mais d'autres types d'alcool peuvent être déshydratés grâce au procédé selon l'invention, notamment le butanol ou le propanol.

Le mélange eau / alcool est vaporisé dans l'unité de vaporisation 3. A ce stade, lorsque l'unité de vaporisation 3 comprend une colonne de distillation, une première séparation entre l'eau et l'alcool a lieu (voir la **figure 1** **bis**). Cette séparation permet de récupérer en bas de colonne de l'eau de désorption (eau pure ou quasi-pure) par la conduite de soutirage d'eau 25. En tête de colonne, on récupère un mélange eau / alcool plus concentré en alcool. Par conséquent, le mélange eau / alcool vaporisé qui transite par la conduite 4 présente une concentration en alcool qui est typiquement comprise entre 80 % et 97 % vol., la borne supérieure de cette gamme étant atteinte lorsqu'une distillation a eu lieu et que le mélange est au point azéotropique. A ce stade, le mélange vaporisé est à une pression comprise entre 1,6 et 2 bars absolus (par exemple à une pression d'environ 1,8 bars absolus) et à une température qui est approximativement la température de vaporisation du mélange.

La vapeur eau / alcool est surchauffée dans le surchauffeur 4'. La température du mélange est élevée à ce stade typiquement de 15 à 45°C, de sorte à dépasser la température de condensation de l'eau. Cette température est donc suffisante pour que le mélange eau / alcool reste à l'état vapeur lors de son passage dans les ballons munis de tamis moléculaire.

### Etape (ii)

Le mélange à l'état vapeur arrive ensuite en tête du ou des ballons (notés 5a à 5c) qui fonctionnent en mode déshydratation, à une température comprise entre 105 et 141°C (notamment d'environ 128°C) et à une pression comprise entre 1,6 et 2 bars absolus (notamment d'environ 1,8 bar absolu) ; il s'écoule au travers du tamis moléculaire selon une direction descendante. En sortie du tamis moléculaire, la vapeur d'alcool déshydraté est à une température comprise entre 100 et 125°C (notamment d'environ 110-115°C) et à une pression dite d'adsorption comprise entre 1,5 et 1,9 bars absolus (notamment d'environ 1,7 bar absolu).

Dans le ballon, le choix de l'écoulement de haut en bas est préféré, puisque, le tamis se chargeant par le haut du ballon, il est ainsi plus facile de remplacer une partie de tamis dégradé, le tamis se dégradant principalement sur les premiers centimètres d'écoulement du fluide à traiter. Au cours du passage au travers des ballons, l'eau du mélange eau / alcool se fixe sur le tamis par adsorption. Ainsi, de l'alcool déshydraté quitte les ballons en question par une ou plusieurs des conduites de soutirage (notées 6a à 6c) qui convergent vers la conduite de soutirage 6.

### Etape (iv)

Mais chaque ballon peut également fonctionner à tour de rôle en mode régénération. En mode régénération, une partie de l'alcool déshydraté est réinjecté dans le ballon de bas en haut. Cette partie de l'alcool déshydraté joue le rôle de fluide de désorption. Le ballon en régénération contenant un tamis chargé en eau a été préalablement dépressurisé par des moyens non représentés à une pression dite pression de désorption, inférieure à la pression atmosphérique et par exemple comprise entre 0,1 et 0,3 bar absolu, notamment égale à environ 0,25 bar absolu. Dans ce ballon, la pression partielle d'eau diminue alors, ce qui provoque sa désorption du tamis, et la vapeur d'eau désorbée est entraînée hors du ballon grâce au courant d'alcool déshydraté. Cet effluent eau / alcool, qui présente une teneur en eau élevée (de l'ordre de 20 à 27 %), quitte alors le ballon par l'une des conduites de soutirage de l'effluent (notées 7a à 7c) puis par la conduite de soutirage principale de l'effluent 7. L'effluent (ou éluat de désorption) est refroidi dans l'unité de refroidissement 8 puis envoyé vers la zone de traitement et / ou de stockage 9. Alternativement, lorsque l'unité de vaporisation 3 comprend une colonne de distillation, il est possible de recycler l'éluat de désorption vers la colonne de distillation via une conduite de recyclage 24 (voir **figure 1** **bis** et **figure 1** **ter**), ce qui permet de récupérer l'alcool contenu dans l'éluat de désorption en le réinjectant dans le circuit de déshydratation.

Il faut noter qu'il est possible de surchauffer le fluide de désorption avant son passage dans le ballon en mode régénération comme cela est par exemple décrit dans le document FR 2 719 039. Ce surchauffage supplémentaire permet un meilleur contrôle de la désorption, notamment en fournissant un paramètre supplémentaire de réglage (à savoir la température de surchauffage supplémentaire) qui assure une meilleure dissociation de l'adsorption et de la régénération. Ce surchauffage supplémentaire permet aussi d'améliorer l'efficacité du tamis moléculaire et de réduire la consommation d'alcool utilisé en tant que gaz de désorption.

Il faut également noter que le nombre de ballons peut être égal à un, deux, trois ou plus.

Plus précisément, dans le cas où l'installation comprend deux ballons, le procédé se décompose en une succession de cycles, dans laquelle un cycle donné comprend une première période et une seconde période : lors de la première période, un premier tamis moléculaire fonctionne d'abord en mode déshydratation d'alcool (adsorption) tandis que le second tamis moléculaire fonctionne en mode régénération (désorption) ; puis, lors de la seconde période, le premier tamis moléculaire fonctionne en mode désorption tandis que le second tamis fonctionne en mode adsorption.

Ce fonctionnement en alternance permet une production d'alcool déshydraté substantiellement en continu, d'où un gain de temps et une optimisation des coûts par rapport au cas, également possible, où le dispositif ne comporte qu'un seul ballon d'adsorption.

Dans le cas, illustré par les diverses figures, où le dispositif comprend trois ballons, chaque tamis moléculaire subit une régénération à tour de rôle, étant entendu que la déshydratation du mélange eau / alcool reste de préférence assurée à tout instant par deux tamis moléculaires sur les trois, avec un décalage du front de désorption entre les deux tamis en fonctionnement.

L'utilisation de trois ou de plus de trois tamis moléculaires est particulièrement adaptée au cas où les durées des phases d'adsorption et de désorption sont différentes.

### Etape (iii)

La vapeur d'alcool déshydraté qui circule dans la conduite de soutirage 6, du moins la partie de celle-ci qui ne sert pas à la régénération des tamis moléculaires, fournit par la suite une part de l'énergie nécessaire à la vaporisation et au surchauffage de l'étape (i) du procédé susmentionné, ce qui constitue un recyclage énergétique.

Selon le mode de réalisation dit de thermo-compression, le transfert énergétique s'effectue au moyen d'un circuit de fluide caloporteur qui, pour les besoins de la présente description, sera de l'eau. En référence à la **figure 1** ou **1** **bis,** la conduite de soutirage 6 apporte la vapeur d'alcool déshydraté dans l'unité de condensation 10, où cette vapeur d'alcool déshydraté se condense (en subissant une chute de température de l'ordre de 5 à 10°C) et cède de l'énergie à l'eau située dans le ballon d'évaporation 12, qui se vaporise. A la suite du passage dans l'unité de condensation 10, l'alcool déshydraté condensé est amené via une conduite de collecte 16 vers le stockage 17 de l'alcool déshydraté.

En sortie du ballon d'évaporation 12, la vapeur d'eau produite est aspirée par le thermo-éjecteur 13, à partir de la source de vapeur à haute pression 14, qui constitue la source mécanique du thermo-éjecteur 13. La vapeur produite dans le ballon 12 a une température comprise entre 84 et 90°C (notamment d'environ 87°C) avec la pression de vapeur correspondante, soit notamment environ 0,62 bar absolu. La source de vapeur à haute pression 14 est fournie à une pression comprise entre 12 et 45 bars absolus, à la température minimum de saturation correspondante, et peut avoir une température de surchauffe de 10 à 40°C. Le thermo-éjecteur alimente ainsi en vapeur comprimée l'échangeur de chaleur 11. En faisant référence à la **figure 1****,** en sortie du thermo-éjecteur la vapeur comprimée est à une pression comprise entre 1 et 1,4 bar absolu (notamment d'environ 1,2 bar absolu) correspondant à une température de vapeur saturée d'environ 105°C. En faisant référence à la **figure 1** **bis,** en sortie du thermo-éjecteur la vapeur comprimée a une pression comprise entre environ 2,25 et 3 bars absolus correspondant à une température de vapeur saturée de 124 à 133°C (notamment d'environ 128°C). La vapeur comprimée se condense en eau liquide dans l'échangeur de chaleur 11 en perdant environ 5 à 10°C, et cède ce faisant de la chaleur au mélange eau / alcool contenu dans l'unité de vaporisation 3, permettant sa vaporisation.

Par ailleurs, le condensat de fluide caloporteur (eau liquide) en sortie de l'échangeur de chaleur 11 est redirigé vers le ballon d'évaporation 12 pour être partiellement re-vaporisé. Le condensat final du fluide caloporteur, partie du condensat intermédiaire qui n'est pas re-vaporisée dans le ballon d'évaporation 12 au moyen de l'unité de condensation 10, est donc considérablement réduite par rapport aux systèmes de l'art antérieur. Selon le rendement du thermo-ejecteur, le pourcentage de condensat re-vaporisé est de 25 à 35 % du condensat produit par l'échangeur de chaleur 11. Ledit condensat final est prélevé par la ligne de sortie 15 issue du ballon d'évaporation 12, où il peut être dirigé vers la chaudière qui fournit la source de vapeur à haute pression 14.

Selon le mode de réalisation particulier représenté à la **figure 1** **ter,** le circuit du fluide caloporteur n'est pas complètement isolé du circuit de déshydratation d'alcool. En effet, dans ce cas l'échangeur de chaleur 11 est omis, et la vapeur d'eau comprimée produite par le thermo-éjecteur 13 est injectée directement dans le soubassement de la colonne de distillation de l'unité de vaporisation 3, dans lequel s'accumule l'eau séparée du mélange eau / alcool par distillation (flegmasses). L'apport de chaleur se fait donc directement dans la colonne, sans utiliser l'intermédiaire de l'échangeur de chaleur 11, ce qui permet une optimisation du rendement énergétique. La conduite de soutirage d'eau 25 en sortie de la partie inférieure de la colonne assure un retour de l'eau vers le ballon d'évaporation 12.

Le transfert d'énergie de la vapeur d'alcool déshydraté vers l'unité de vaporisation 3 peut également s'effectuer selon un autre mode de réalisation, dit de compression mécanique (voir **figure 2** et **figure 2** **bis**).

Selon ce mode de réalisation, la vapeur d'alcool déshydraté issue de la conduite de soutirage 6 passe dans une unité de compression mécanique 18. Lors de ce passage, la pression de la vapeur d'alcool déshydraté augmente. La compression des vapeurs d'alcool permet d'augmenter la température de vapeur saturante d'environ 8°C, soit de passer d'une température de vapeur saturante comprise entre 89 et 95°C à une température de vapeur recomprimée correspondant à une température de vapeur saturante entre 97 et 103°C. La compression des vapeurs permet ainsi de passer d'une pression d'environ 1,5 à 2 bar abs., à une pression d'environ 2,05 à 2,55 bar abs. Les vapeurs d'alcool déshydraté avant recompression ont une surchauffe comprise entre 10 et 30°C. Ces vapeurs sont partiellement désurchauffées avant compression pour respecter les contraintes de tenue mécanique du compresseur. Le compresseur mécanique peut être du type « Roots » ou « Soufflante » à plusieurs étages. La vapeur ainsi recomprimée circule dans la conduite intermédiaire 19 et peut être utilisée pour évaporer (et surchauffer) directement en partie le mélange eau / alcool lors de l'étape (i), sans devoir utiliser de fluide caloporteur intermédiaire, simplement par condensation de la vapeur d'alcool recomprimée dans l'unité de condensation 20. Les vapeurs d'alcool déshydraté sont condensées à une température comprise entre 95 et 102°C à la pression d'équilibre correspondante. Tout comme dans le cas de la compression thermique, l'alcool déshydraté condensé, au sortir de l'unité de condensation 20, est récupéré par la conduite de collecte 16 et amené vers l'unité suivante de stockage ou de traitement 17.

Comme la chaleur transférée au mélange eau / alcool au niveau de l'unité de condensation 20 ne suffit pas à assurer la vaporisation complète du mélange eau / alcool de l'étape (i), un apport complémentaire d'énergie est assuré au niveau de l'échangeur de chaleur 21 par la condensation d'une vapeur auxiliaire (à basse pression) 22, qui est en général de la vapeur d'eau, mais pourra également être toute autre forme de vapeur. Le condensat résultant de la condensation de la vapeur auxiliaire est récupéré dans la conduite 23.

Le circuit auxiliaire en question peut alimenter directement en énergie l'unité de vaporisation 3 via un contact entre l'échangeur de chaleur 21 et l'unité de vaporisation 3, comme c'est le cas par exemple dans le mode de réalisation de la **figure 2****,** ou bien cette alimentation énergétique peut être indirecte, comme c'est le cas par exemple dans le mode de réalisation de la **figure 2** **bis.** En effet, selon cette seconde variante, le circuit auxiliaire chauffe la colonne de distillation 26 qui, étant alimentée en éluat de désorption par la conduite de recyclage 24, assure une séparation de l'eau et de l'alcool de cet éluat de désorption. Le mélange eau / alcool vaporisé issu de la colonne de distillation 26, reconcentré en alcool par rapport à l'éluat de désorption, est acheminé via la conduite 27 vers l'échangeur de chaleur 28, où il se condense et permet de chauffer partiellement l'unité de vaporisation 3. Le condensat de mélange eau / alcool en sortie de l'échangeur 28 est orienté par la conduite 29 pour partie en retour vers la colonne de distillation 26, et pour partie vers l'unité de vaporisation 3. Ainsi, dans ce mode de réalisation (tout comme d'ailleurs dans les modes de réalisation de thermo-compression représentés à la **figure 1** **bis** et à la **figure 1** **ter**), le recyclage énergétique permis par l'invention se double d'un recyclage matériel de l'éluat de désorption, dont la portion alcoolique est récupérée.

L'alcool déshydraté obtenu grâce au procédé selon l'invention, dans ses divers modes de réalisation, présente un degré de pureté typique de 99,9 % en volume.

Il reste entendu que lors d'une phase d'initiation du procédé, c'est à dire avant que l'on ait commencé à obtenir de l'alcool déshydraté dont l'énergie thermique est susceptible d'être recyclée, seuls les moyens complémentaires assurant la vaporisation et le surchauffage du mélange eau / alcool sont en fonctionnement.

### EXEMPLES

Un exemple d'installation selon l'invention conformément au mode de réalisation de la **figure 1** **bis** a été mis en oeuvre. Le produit à déshydrater est de l'alcool brut à 93 % vol. L'installation fonctionne à partir de vapeur haute pression à 22 bar effectifs. Cet exemple a permis de réduire la consommation de vapeur à 40 kg pour 100 litres d'alcool pur total obtenu, contre 60 kg pour 100 litres dans le cas d'un procédé classique de déshydratation sur tamis moléculaire à partir de vapeur basse pression à 2 bar effectifs. La consommation électrique de l'installation est de 25 kW.

Un autre exemple d'utilisation selon l'invention conformément au mode de réalisation représenté à la **figure 2** **bis** a également été mis en oeuvre. Cette installation utilise 35 kg de vapeur basse pression à 4 bar effectifs pour 100 litres d'alcool pur total obtenu et présente une consommation électrique de 140 kW.

Dans l'un et l'autre de ces exemples, la capacité moyenne de production est d'environ 200 m³ d'alcool déshydraté par jour.

## Revendications

1. Procédé de déshydratation d'un mélange eau / alcool comprenant les étapes suivantes :
(i) vaporisation et surchauffe du mélange eau / alcool, à une température suffisante pour maintenir ledit mélange à l'état vapeur au cours de l'étape (ii) ;
(ii) adsorption par passage du mélange eau / alcool à l'état vapeur obtenu à l'étape (i) sur un tamis moléculaire pour provoquer l'adsorption de l'eau sur ledit tamis moléculaire, permettant d'obtenir de la vapeur d'alcool déshydraté ;
(iii) condensation de la vapeur d'alcool déshydraté obtenue à l'étape (ii), permettant de récupérer de l'énergie, dans lequel la vaporisation et / ou la surchauffe du mélange eau / alcool de l'étape (i) sont effectuées au moins partiellement grâce à l'énergie récupérée à l'étape (iii) ; et
dans lequel :
- la condensation de l'étape (iii) est précédée d'une compression mécanique de la vapeur d'alcool déshydraté ; ou
- la condensation de l'étape (iii) est couplée à la vaporisation d'un fluide caloporteur et le fluide caloporteur vaporisé subit une compression.

2. Procédé selon la revendication 1, dans lequel l'alcool est l'éthanol.

3. Procédé selon l'une des revendications 1 ou 2, dans lequel le mélange eau / alcool contient au moins 80 % en volume d'alcool, de préférence au moins 90 % en volume d'alcool, de préférence au moins 92 % en volume d'alcool.

4. Procédé selon l'une des revendications 1 à 3, comprenant, concurremment et / ou alternativement avec les étapes (i) à (iii), l'étape supplémentaire suivante :
(iv) régénération par passage d'une partie de l'alcool déshydraté, à une pression inférieure à la pression atmosphérique, sur un tamis moléculaire saturé en eau pour désorber l'eau adsorbée,
ladite étape produisant un effluent.

5. Procédé selon la revendication 4, comprenant à l'étape (iv) un surchauffage de la partie de l'alcool déshydraté devant passer sur le tamis moléculaire saturé en eau pour désorber l'eau adsorbée.

6. Procédé selon la revendication 4 ou 5, dans lequel l'effluent obtenu à l'étape (iv) est combiné avec le mélange eau / alcool à traiter.

7. Procédé selon l'une des revendications 1 à 6, dans lequel deux, trois tamis moléculaires ou plus opèrent selon un mode en alternance.

8. Procédé selon l'une des revendications 1 à 7, nécessitant une consommation de vapeur d'eau inférieure à environ 50 kg pour 100 litres d'alcool déshydraté produit.

9. Installation pour la déshydratation d'un mélange eau / alcool, comprenant :
- une conduite d'amenée (2) du mélange eau / alcool à traiter ;
- une unité de vaporisation (3) pour assurer la vaporisation du mélange circulant dans la conduite précitée ;
- un surchauffeur (4') pour surchauffer la vapeur issue de l'unité de vaporisation (3) ;
- un ou plusieurs ballons (5a, 5b, 5c) connectés au surchauffeur (4') et contenant un tamis moléculaire ;
- une unité de condensation (10, 20) de l'alcool déshydraté, en aval des ballons (5a, 5b, 5c) et assurant une partie de l'alimentation en énergie de l'unité de vaporisation (3) ;
- une ou des conduites de collecte de l'alcool déshydraté (16) connectées en sortie de l'unité de condensation (10, 20) ;
et comprenant en outre :
- une unité de compression mécanique (18) entre les ballons (5a, 5b, 5c) et l'unité de condensation (20) ; ou
- un circuit de fluide caloporteur, ledit circuit étant prévu pour permettre les échanges de chaleur entre le fluide caloporteur et, d'une part l'alcool déshydraté au niveau de l'unité de condensation (10), d'autre part l'eau ou le mélange eau / alcool au niveau de l'unité de vaporisation (3) au moyen d'une injection directe ou au moyen d'un échangeur de chaleur (11), le circuit de fluide caloporteur comprenant un système de thermo-éjection (13) alimenté en vapeur à haute pression (14) prévu pour recomprimer le fluide caloporteur à l'état vapeur.

10. Installation selon la revendication 9, dans laquelle l'unité de vaporisation (3) comprend une colonne de distillation.

11. Installation selon la revendication 9 ou 10, dans laquelle l'unité de condensation (20) est prévue pour évaporer directement le mélange eau / alcool au niveau de l'unité de vaporisation (3).

12. Installation selon l'une des revendications 9 à 11, comprenant en outre un échangeur de chaleur (21) assurant une partie de l'alimentation en énergie de l'unité de vaporisation (3).

13. Installation selon l'une des revendications 9 à 12, comprenant en outre :
- des conduites de désorption pour ramener une partie de l'alcool déshydraté vers les ballons ;
- des conduites de soutirage (7a, 7b, 7c, 7) de l'effluent issu des ballons (5a, 5b, 5c) après régénération.

14. Installation selon la revendication 13, comprenant en outre une conduite de recyclage (24) destinée à recycler l'effluent vers l'unité de vaporisation (3).

15. Installation selon la revendication 13, comprenant en outre une conduite de recyclage (24) destinée à recycler l'effluent vers une unité de distillation (26) alimentant en vapeur un échangeur de chaleur (28) destiné à fournir de l'énergie à l'unité de vaporisation (3).

16. Installation selon l'une des revendications 13 à 15, comprenant en outre un moyen de chauffage placé au contact des conduites de désorption.

## Patentansprüche

1. Verfahren zur Dehydratisierung eines Wasser/Alkohol-Gemischs, das die folgenden Schritte umfasst:
(i) Verdampfen und Überhitzen des Wasser/Alkohol-Gemischs auf eine Temperatur, die ausreicht, um das Gemisch im Verlauf von Schritt (ii) im Dampfzustand halten;
(ii) Adsorbieren durch Durchleiten des in Schritt (i) erhaltenen Wasser/Alkohol-Gemisch im Dampfzustand durch ein Molekularsieb, um die Adsorption des Wassers auf dem Molekularsieb hervorzurufen, wodurch es möglich ist, dehydratisierten Alkoholdampf zu erhalten;
(iii) Kondensieren des in Schritt (ii) erhaltenen dehydratisierten Alkoholdampfs, wodurch es möglich ist, Energie zurückzugewinnen,
wobei das Verdampfen und/oder das Übererhitzen des Wasser/Alkohol-Gemischs aus Schritt (i) mindestens teilweise mithilfe der in Schritt (iii) zurückgewonnenen Energie durchgeführt wird, und wobei:
- dem Kondensieren aus Schritt (iii) eine mechanische Verdichtung des dehydratisierten Alkoholdampfs vorausgeht; oder
- das Kondensieren aus Schritt (iii) mit dem Verdampfen eines Wärmeträgermediums gekoppelt ist und das verdampfte Wärmeträgermedium komprimiert wird.

2. Verfahren nach Anspruch 1, wobei es sich bei dem Alkohol um Ethanol handelt.

3. Verfahren nach einem der Ansprüche 1 oder 2, wobei das Wasser/Alkohol-Gemisch mindestens 80 Vol.-% Alkohol, bevorzugt mindestens 90 Vol.-% Alkohol, bevorzugt mindestens 92 Vol.-% Alkohol enthält.

4. Verfahren nach einem der Ansprüche 1 bis 3, das gleichzeitig und/oder alternativ zu den Schritten (i) bis (iii) den folgenden zusätzlichen Schritt umfasst:
(iv) Regenerieren durch Durchleiten eines Teils des dehydratisierten Alkohols bei einem Druck unterhalb des Atmosphärendrucks auf ein mit Wasser gesättigtes Molekularsieb, um das adsorbierte Wasser zu desorbieren,
wobei bei diesem Schritt ein Abfluss erzeugt wird.

5. Verfahren nach Anspruch 4, das in Schritt (iv) ein Überhitzen des dehydratisierten Alkoholanteils umfasst, bevor er durch das mit Wasser gesättigte Molekularsieb geleitet wird, um das adsorbierte Wasser zu desorbieren.

6. Verfahren nach Anspruch 4 oder 5, wobei der in Schritt (iv) erhaltene Abfluss mit dem zu behandelnden Wasser/Alkohol-Gemisch kombiniert wird.

7. Verfahren nach einem der Ansprüche 1 bis 6, wobei zwei, drei oder mehr Molekularsiebe in einem alternierenden Modus arbeiten.

8. Verfahren nach einem der Ansprüche 1 bis 7, das einen Wasserdampfverdampfverbrauch von weniger als etwa 50 kg je 100 Liter erzeugtem dehydratisiertem Alkohol benötigt.

9. Anlage zur Dehydratisierung eines Wasser/Alkohol-Gemischs, umfassend:
- eine Zuführleitung (2) für das zu behandelnde Wasser/Alkohol-Gemisch;
- eine Verdampfungseinheit (3), um das Verdampfen des Gemischs zu bewirken, das in der vorgenannten Leitung zirkuliert;
- einen Überhitzer (4'), um den aus der Verdampfungseinheit (3) stammenden Dampf zu überhitzen;
- eine oder mehrere Kolben (5a, 5b, 5c), die mit dem Überhitzer (4') verbunden sind und ein Molekularsieb enthalten;
- eine Einheit (10, 20) zum Kondensieren des dehydratisierten Alkohols stromabwärts der Kolben (5a, 5b, 5c), die auch einen Teil der Versorgung der Verdampfungseinheit (3) mit Energie sicherstellt;
- eine oder mehrere Leitungen zum Sammeln des dehydratisierten Alkohols (16), die mit dem Auslass der Kondensationseinheit (10, 20) verbunden ist bzw. sind;
und ferner umfassend:
- eine Einheit zum mechanischen Komprimieren (18) zwischen den Kolben (5a, 5b, 5c) und der Kondensationseinheit (20); oder
- einen Wärmeträgermediumkreislauf, wobei der Kreislauf vorgesehen ist, um den Wärmeaustausch zwischen dem Wärmeträgermedium und dem dehydratisierten Alkohol in der Kondensationseinheit (10) einerseits, dem Wasser oder Wasser/Alkohol-Gemisch in der Verdampfungseinheit (3) andererseits mithilfe einer direkten Injektion oder mithilfe eines Wärmetauschers (11) zu ermöglichen, wobei der Wärmeträgermediumkreislauf ein Thermoejektionssystem (13) umfasst, das mit Hochdruckdampf (14) versorgt wird, der dazu bestimmt ist, das Wärmeträgermedium im Dampfzustand rückzuverdichten.

10. Anlage nach Anspruch 9, wobei die Verdampfungseinheit (3) eine Destillationssäule umfasst.

11. Anlage nach Anspruch 9 oder 10, wobei die Kondensationseinheit (20) vorgesehen ist, um das Wasser/Alkohol-Gemisch in der Verdampfungseinheit (3) direkt zu verdampfen.

12. Anlage nach einem der Ansprüche 9 bis 11, die ferner einen Wärmetauscher (21) umfasst, der einen Teil der Energieversorgung der Verdampfungseinheit (3) sicherstellt.

13. Anlage nach einem der Ansprüche 9 bis 12, die ferner umfasst:
- Desorptionsleitungen, um einen Teil des dehydratisierten Alkohols in die Kolben zurückzuleiten;
- Leitungen (7a, 7b, 7c, 7) zur Entnahme des aus den Kolben (5a, 5b, 5c) stammenden Abflusses nach der Regeneration.

14. Anlage nach Anspruch 13, die ferner eine Rückführungsleitung (24) umfasst, die dazu bestimmt ist, den Abfluss in die Verdampfungseinheit (3) zurückzuführen.

15. Anlage nach Anspruch 13, die ferner eine Rückführungsleitung (24) umfasst, die dazu bestimmt ist, den Abfluss in eine Destillationseinheit (26) zurückzuführen, die einen Wärmetauscher (28) mit Dampf versorgt, der dazu bestimmt ist, Energie an die Verdampfungseinheit (3) zu liefern.

16. Anlage nach einem der Ansprüche 13 bis 15, die ferner ein Mittel zum Erhitzen umfasst, das in Kontakt mit den Desorptionsleitungen angeordnet ist.

## Claims

1. A method for dehydrating a water/alcohol mixture comprising the following steps:
(i) vaporizing and overheating the water/alcohol mixture, at a sufficient temperature for maintaining said mixture in the vapor state during step (ii);
(ii) adsorbing by having the water/alcohol mixture in the vapor state obtained in step (i) pass over a molecular sieve in order to cause adsorption of the water on said molecular sieve, whereby a dehydrated alcohol vapor is obtained;
(iii) condensing the dehydrated alcohol vapor obtained in step (ii), whereby energy is recovered,
wherein the vaporisation and/or the overheating of the water/alcohol mixture of step (i) are at least partly carried out by means of the energy recovered in step (iii); and wherein:
- the condensation of step (iii) is preceded by mechanical compression of the dehydrated alcohol vapor; or
- the condensation of step (iii) is coupled with the vaporization of a heat transfer fluid and the vaporized heat transfer fluid is subject to compression.

2. The method according to claim 1, wherein the alcohol is ethanol.

3. The method according to one of claims 1 or 2, wherein the water/alcohol mixture contains at least 80% by volume of alcohol, preferably at least 90% by volume of alcohol, preferably at least 92% by volume of alcohol.

4. The method according to one of claims 1 to 3, comprising, concurrently and/or alternatively with steps (i) to (iii), the following additional step:
(iv) regeneration by the passing of a portion of the dehydrated alcohol, to a pressure below atmospheric pressure, over a molecular sieve saturated with water in order to desorb the adsorbed water,
said step producing an effluent.

5. The method according to claim 4, comprising in step (iv) overheating of the portion of the dehydrated alcohol which has to pass over the molecular sieve saturated with water for desorbing the adsorbed water.

6. The method according to claim 4 or 5, wherein the effluent obtained in step (iv) is combined with the water/alcohol mixture to be treated.

7. The method according to one of claims 1 to 6, wherein two, three molecular sieves or more operate according to an alternating method.

8. The method according to one of claims 1 to 7, requiring water vapor consumption of less than about 50 kg for 100 liters of produced dehydrated alcohol.

9. A facility for dehydrating a water/alcohol mixture, comprising:
- a conduit (2) for supplying the water/alcohol mixture to be treated;
- a vaporisation unit (3) for ensuring a vaporisation of the mixture circulating in the aforementioned conduit;
- an overheater (4') for overheating the vapor from the vaporisation unit (3);
- one or more flasks (5a, 5b, 5c) connected to the overheater (4') and containing a molecular sieve;
- a unit (10, 20) for condensing the dehydrated alcohol, downstream from the flasks (5a, 5b, 5c) and ensuring a portion of the power supply of the vaporization unit (3);
- conduit(s) (16) for collecting the dehydrated alcohol connected at the outlet of the condensation unit (10, 20);
and further comprising:
- a mechanical compression unit (18) between the flasks (5a, 5b, 5c) and the condensation unit (20); or
- a heat transfer fluid circuit, said circuit being provided for allowing heat exchanges between the heat transfer fluid and the dehydrated alcohol at the condensation unit (10) on the one hand, the water or the water/alcohol mixture at the vaporization unit (3) by means of direct injection or by means of a heat exchanger (11) on the other hand, the heat transfer fluid circuit comprising a thermo-ejection system (13) fed with high pressure vapor (14) provided for re-compressing the heat transfer fluid in the vapor state.

10. The facility according to claim 9, wherein the vaporization unit (3) comprises a distillation column.

11. The facility according to claim 9 or 10, wherein the condensation unit (20) is provided for directly evaporating the water/alcohol mixture at the vaporisation unit (3).

12. The facility according to one of claims 9 to 11, further comprising a heat exchanger (21) ensuring a portion of the power supply of the vaporization unit (3).

13. The facility according to one of claims 9 to 12, further comprising:
- desorption conduits for bringing back a portion of the dehydrated alcohol towards the flasks;
- conduits (7a, 7b, 7c, 7) for drawing off the effluent from the flasks (5a, 5b, 5c) after regeneration.

14. The facility according to claim 13, further comprising a recycling conduit (24) intended for recycling the effluent towards the vaporisation unit (3).

15. The facility according to claim 13, further comprising a recycling conduit (24) intended for recycling the effluent towards a distillation unit (26) feeding with vapor a heat exchanger (28) intended to provide energy to the vaporization unit (3).

16. The facility according to one of claims 13 to 15, further comprising a heating means placed in contact with the desorption conduits.
